# EUROPEAN PATENT SPECIFICATION

(11) **EP 1 729 746 B1**
(45) Date of publication and mention of the grant of the patent: **18.11.2009**
(21) Application number: 05708757.9
(22) Date of filing: 10.03.2005
(51) Int. Cl.: A61K 9/70, A61K 31/465, A61K 9/00

(54) **MEANS FOR TRANSDERMAL ADMINISTRATION OF NICOTINE**
MITTEL FÜR DIE TRANSDERMALE VERABREICHUNG VON NIKOTIN
DISPOSITIF D'ADMINISTRATION TRANSDERMIQUE DE LA NICOTINE

(30) Priority: 19.03.2004 SE 0400685; 02.04.2004 US 558874 P
(43) Date of publication of application: 13.12.2006
(73) Proprietor: McNeil AB, 254 42 Helsingborg (SE)
(72) Inventor: LINDELL, Eva, Anette, Katarina, S-251 09, Helsingborg (SE); NICKLASSON, Per, Fredrik, S-251 09, Helsingborg (SE); THYRESSON, Maarit, Kristina, S-251 09, Helsingborg (SE)
(74) Representative: Hedenström, John Allan
(86) International application number: PCT/IB2005/000673
(87) International publication number: WO 2005/089728

(56) References cited:
- WO-A-96/00111
- US-A- 5 415 629
- US-A- 5 505 957
- US-A- 5 721 257
- US-A1- 2001 033 858

## Description

### Technical Field

The present invention relates to devices for transdermal administration of nicotine, specifically such devices providing for basic as well as additional user activatable administration of nicotine. The present invention also relates to use of said devices.

### Background of the Invention

### Tobacco dependence and reduction thereof

In recent years, with the recognition of the harmful effects of tobacco smoking, there have been numerous campaigns and programs by governmental agencies and various health groups and other interested organisations to disseminate information about the adverse health effects resulting from tobacco smoking. Moreover, and as a result of this recognition of the harmful effects, there have been many programs directed to attempts in reducing smoking incidence.

Nicotine is an organic compound and is the principal alkaloid of tobacco. Nicotine is the chief addictive ingredient in the tobacco used in cigarettes, cigars, snuff and the like. Nicotine is also an addictive drug, though, and smokers characteristically display a strong tendency to relapse after having successfully stopped smoking for a time. Nicotine is the worlds second most used drug, after caffeine from coffee and tea.

The main problem with tobacco smoking is its enormous implications on health. It is estimated that smoking related diseases cause some 3-4 million deaths per year globally. According to Centers for Disease Control and Prevention. Cigarette smoking among adults - United States, 1995. MMWR 1997;46:1217-1220 around 500,000 persons in USA die each year as a result of tobacco use. In fact, excessive smoking is now recognised as one of the major health problems throughout the world. This grim consequence of tobacco smoking has urged many medical associations and health authorities to take very strong actions against the use of tobacco.

Even though tobacco smoking is decreasing in many developed countries today it is hard to see how the societies could get rid of the world's second most used drug.

The most advantageous thing a heavy smoker can do is to reduce or preferably even stop smoking completely. Experience shows, however, that most smokers find this extremely difficult since, mostly, tobacco smoking results in a dependence disorder or craving. The WHO has in its International Classification of Disorders a diagnosis called Tobacco Dependence. Others like the American Psychiatric Association call the addiction Nicotine Dependence. It is generally accepted that these difficulties to stop smoking result from the fact those heavy smokers are dependent on nicotine. The most important risk factors are, however, substances that are formed during the combustion of tobacco, such as carbon monoxide, tar products, aldehydes, and hydro cyanic acid.

### Effects of nicotine

The administration of nicotine can give satisfaction and the usual method is by smoking, either by smoking e g a cigarette, a cigar or a pipe. However, smoking has health hazards and it is therefore desirable to formulate an alternative way of administering nicotine in a pleasurable manner that can be used to facilitate withdrawal from smoking and/or used as a replacement for smoking.

When smoking a cigarette, nicotine is quickly absorbed into the smoker's blood and reaches the brain within around ten seconds after inhalation. The quick uptake of nicotine gives the consumer a rapid satisfaction, or kick. The satisfaction, then, lasts during the smoking time of the cigarette and for a period of time thereafter. The poisonous, toxic, carcinogenic, and addictive nature of smoking has provided efforts for methods, compositions and devices, which help in breaking the habit of smoking cigarettes.

Nicotine is an addictive poisonous alkaloid C₅H₄NC₄H₇NCH₃, derived from the tobacco plant. Nicotine is also used as an insecticide.

### Nicotine replacement products

One way to reduce smoking is to provide nicotine in a form or manner other than by smoking and some products have been developed to fulfil this need. Nicotine containing formulations are currently the dominating treatments for tobacco dependence.

The successes in achieving reduction in the incidence of smoking have been relatively poor using presently known products. The present state of the art involves both behavioural approaches and pharmacological approaches. More than 80 % of the tobacco smokers who initially quit smoking after using some behavioural or pharmacological approach to singly reduce smoking incidence generally relapse and return to the habit of smoking at their former rate of smoking within about a one year's period of time.

As an aid for those who are willing to stop smoking there are several ways and forms of nicotine replacement products available on the market, such as nicotine chewing gum. Several methods and means have been described for diminishing the desire of a subject to use tobacco, which comprises the step of administering to the subject nicotine or a derivative thereof as described in e g US 5,810,018 (oral nicotine spray), US 5,939,100 (nicotine containing microspheres) and US 4,967,773 (nicotine containing lozenge).

Nicotine-containing nose drops have been reported (Russell et al., British Medical Journal, Vol. 286, p. 683 (1983); Jarvis et al., Brit. J. of Addiction, Vol. 82, p. 983 (1987)). Nose drops, however, are difficult to administer and are not convenient for use at work or in other public situations. Ways of administrating nicotine by way of delivering directly into the nasal cavity by spraying is known from US 4,579,858, DE 32 41437 and WO93/127 64. There may, though, be local nasal irritation with use of nasal nicotine formulations. The difficulty in administration also results in unpredictability of the dose of nicotine administered.

Also, inhaling devices resembling a cigarette are known for uptake of nicotine vapours as suggested in US 5,167,242.

One successful approach to date in reducing the incidence of smoking relies upon nicotine containing chewing gum that is designed to reduce smoking withdrawal symptoms. The reported success rate is approximately twice that of placebo.

One successful product that is used as a smoking substitute and/or as a smoking cessation aid and which is based on nicotine, is the chewing gum Nicorette^{®}. This product was one of the first nicotine replacement forms that was approved by the Food and Drug Administration (FDA) and is still one of the most used nicotine replacement products. Nicorette^{®} chewing gum has been on the market in about 60 countries for several years. In this chewing gum the nicotine is present in the form of a complex with an insoluble cation-exchanger (polacrilex) that is dispersed in a gum base. The nicotine is slowly released from the gum due to chewing and will reach similar plasma levels as when smoking a cigarette after about 30 minutes depending on the chewing technique, i e slow or active chewing. Patents related to this product are e g US Patents 3,877,468, 3,901,248 and 3,845,217.

### Nicotine transdermal patches

The use of skin patches for transdermal administration of nicotine was reported many years ago (Rose, in Pharmacologic Treatment of Tobacco Dependence, (1986) pp. 158-166, Harvard Univ. Press). A large number of patents on devices for transdermal delivery of nicotine have been issued, e g US 5,120,546 disclosing a transdermal delivery system wherein the nicotine is complexed to a cyclo compound, US 5,230,896 disclosing a transdermal delivery system wherein is made use of an acrylic polymer adhesive, US 4,943,435 disclosing a transdermal patch for delivering nicotine for 12 - 24 hours making use of a rate-controlling membrane, US 4,915,950 disclosing a method for making nicotine-containing transdermal delivery devices whereby the nicotine is printed onto an adsorbent fabric layer, US 5,533,995 disclosing a transdermal device wherein the nicotine transport within the device as such is controllable using an internal electrode system, US 5,135,753 disclosing a transdermal device for administering nicotine combined with a nicotine-containing lozenge, US 5,721,257 disclosing a transdermal device for administering nicotine combined with a nasal spray for administering nicotine, WO 0164149 disclosing use of a device for transdermally administering nicotine in combination with heat, and WO 9600111 disclosing transdermal delivery of a drug, e g nicotine, using electrical pulses.

### Prior art and problems thereof

There are known methods for providing an essentially constant base-line plasma level of nicotine being supplemented with boost doses of nicotine when the patient so desires. These methods to date imply use of two different dosage forms for delivery of nicotine to be used in combination. For this purpose may e g be used a nicotine patch in combination with a nicotine lozenge, see US 5,135,753, or a nicotine patch in combination with a nicotine nasal spray, see US 5,721,257 US 5,415,629 discloses a device comprising iontophoretic and ultrasonic delivery. US 5,721,257 discloses use of a transdermal patch in combination with transmucosal administration. For convenience of the user there is a need to provide a single dosage form, which may provide an essentially constant base-line plasma level of nicotine as well as additional boost doses of nicotine when the patient/user so desires.

The present invention pertains to a solution to the captioned problem by providing a unitary device, which accounts for a basic as well as an additional user activatable transdermal delivery of nicotine.

It is highly desirable in light of the aforementioned problems to develop means and methods for the administration of nicotine to provide satisfaction to a person craving for nicotine or to provide a sense of smoking satisfaction without smoking, which can also avoid problems associated with the prior art means and methods. In this respect, the present invention addresses this need and interest.

### Definitions

With "basic transdermal administration of nicotine in any form " and similar expressions is herein meant transdermal nicotine transport provided by a part of the claimed device that administers nicotine continuously during the period of the intended application of the claimed device on the skin.

With "additional transdermal administration of nicotine in any form " and similar expressions is herein meant transdermal nicotine transport provided by a part of the claimed device that at user activation administers additional nicotine, beyond that provided by the part of the claimed device providing for basic administration of nicotine in any form.

### Legend of Figures

Figure 1 is a schematic drawing of the embodiment according to Example 1.
Figure 2 is a schematic drawing of the embodiment according to Example 2.
Figure 3 is a schematic drawing of the embodiment according to Example 3.
Figure 4 is a schematic drawing of the embodiment according to Example 4.
Figure 5 is a schematic drawing of the embodiment according to Example 5.
Figure 6 is a schematic drawing of the embodiment according to Example 6.

### Summary of the Invention

In view of the foregoing disadvantages known in the art when trying to deliver nicotine to a subject to provide different delivery rates of nicotine to a subject the present invention provides a device according to claim 1 for combined basic and additional user activatable transdermal delivery of nicotine.

Further, the present invention provides use of nicotine for the manufacture of a device, and a method for aiding in smoking cessation, in temporary smoking abstinence and/or in reducing the urge to smoke or to otherwise use tobacco containing material, and/or for treating conditions suitable for treatment with nicotine, such conditions being selected from the group consisting of Alzheimer's disease, Crohn's disease, Parkinson's disease, Tourette's syndrome, ulcerous colitis and post-smoking-cessation weight control by transdermal administration of nicotine with such a device. The method of transdermal delivery of nicotine can also be practiced in combination with means for delivering of nicotine selected from mouth sprays, nasal sprays, transdermal patches, inhaling devices, lozenges, tablets and from parenteral methods, subcutaneous methods, intravenous methods, rectal methods, vaginal methods and transmucousal methods, including use of tobacco.

### Categorization of transdermal devices

Means for transdermal administration of nicotine in any form can be categorized in many different ways. A comprehensive categorization of transdermal devices useful in the present invention is in four main groups as follows:
- the reservoir type, in which the drug is placed in a liquid or a gel and delivered across a rate-moderating membrane to the skin;
- the matrix type, in which the drug is placed within a non-adhesive polymeric material, typically a hydrogel or soft polymer;
- the drug-in-adhesive type, in which the drug is placed within an adhesive polymer;
- the multi-laminate type, which is similar to the drug-in-adhesive design, but which incorporates an additional layer of pressure sensitive adhesive to cover the entire device and affix it to the skin. A membrane can also be incorporated into the multi-laminate type.

### User activatable delivery of nicotine

Means for modifying transdermal flux of pharmaceutically active substances can be categorized in many different ways. One such categorization is according to below Table 2.

Since many of the means listed in Table 2 may be designed to be both activated and deactivated by the user, they may be well suited to be used for additional transdermal administration of nicotine in the present invention. Means especially suited for user activation and deactivation, and thus preferred for use in additional transdermal administration of nicotine of the present invention are iontophoresis (including electromigration and electroosmosis), sonophoresis, micro-needles, jet injection or combinations thereof.

**Table 2. Means for modifying transdermal delivery**

| **Means** | **Mechanism** |
|---|---|
| Iontophoresis (electromigration, electroosmosis) | Transport of charged drug molecules in an electrical field drives drug molecules through the Stratum Corneum (SC), enhancing transdermal transport rate. |
| Electroporation | Transport-enhancing pores through SC created by an electrical current. |
| Sonophoresis | Transport enhancement by heating and/or disordering SC by means of ultrasound. |
| Micro-needles | Microscopic needles, loaded with drug, punctures the SC thus increasing transdermal drug flux. |
| Chemical enhancers | Chemicals interacting with the structure of SC, leading to increased permeability of drug molecules through SC. |
| Carrier particles (e.g. transfersomes, lipopearls) | Drug enveloped in submicron particles with good SC permeability. |
| Jet injection | Rapidly expanding gas "shoots" drug particles through SC, leading to increased permeability of drug molecules. |
| Laser microporation | Laser light punctures SC, leading to increased permeability of drug molecules. |
| Tape stripping | Adhesive tape strips off SC, leading to increased permeability of drug molecules. |
| Suction ablation | Suction device strips off SC, leading to increased permeability of drug molecules. |
| Metabolic inhibitors | Chemicals that slows the reparative processes of the skin. Damaged SC more permeable to drug than undamaged SC. |
| Supersaturation | Maximizes the concentration gradient at the drug/SC interface thus optimizing transdermal diffusion of drug. |
| Occlusion | Transport enhancement through heating and/or disordering SC by means of an occlusive dressing. |
| Solvent drag | The transdermal flux of a highly permeable substance drags drug molecules along with it, thus increasing the transdermal transport rate of the drug. |

The means of Table 2 are as such known in the art.

### The active ingredient

According to the invention, the claimed device comprises nicotine in any form.

Nicotine is intended to include nicotine, 3-(1-methyl-2-pyrrolidinyl)-pyridine, with its base form, including synthetic nicotine as well as nicotine extracts from tobacco plants, or parts thereof, such as the genus Nicotiana alone or in combination.

Nicotine in any form is selected from the group consisting of a nicotine salt, the free base form of nicotine, a nicotine derivative, such as a nicotine cation exchanger, a nicotine inclusion complex or nicotine in any non-covalent binding; nicotine bound to zeolites; nicotine bound to cellulose or nicotine bound to starch microspheres; and mixtures thereof.

Numerous nicotine salts are known, and may be used, e g the salts presented in below Table 3.

**Table 3 Acids useful for nicotine salt formation**

| Acid | Molar ratio* of acid:nicotine |
|---|---|
| Formic | 2:1 |
| Acetic | 3:1 |
| Propionic | 3:1 |
| Butyric | 3:1 |
| 2-Methylbutyric | 3:1 |
| 3-Methylbutyric | 3:1 |
| Valeric | 3:1 |
| Lauric | 3:1 |
| Palmitic | 3:1 |
| Tartaric | 2:1 |
| Citric | 2:1 |
| Malic | 2:1 |
| Oxalic | 2:1 |
| Benzoic | 1:1 |
| Gentisic | 1:1 |
| Gallic | 1:1 |
| Phenylacetic | 3:1 |
| Salicylic | 1:1 |
| Phthalic | 1:1 |
| Picric | 2:1 |
| Sulfosalicylic | 1:1 |
| Tannic | 1:5 |
| Pectic | 1:3 |
| Alginic | 1:2 |
| Hydrochloric | 2:1 |
| Chloroplatinic | 1:1 |
| Silicotungstic | 1:1 |
| Pyruvic | 2:1 |
| Glutamic | 1:1 |
| Aspartic | 1:1 |

| | |
|---|---|
| *recommended upon production | |

The above-mentioned cation exchanger is preferably a polyacrylate.

The above-mentioned inclusion complex is preferably a complex with a cyclodextrin, such as β-cyclodextrin.

The above-mentioned nicotine salt is preferably a tartrate, hydrogen tartrate, citrate, maleate or hydrochloride.

The form of nicotine being provided through the basic administration may be another form of nicotine than the one being provided through the additional administration.

### Further additives

Further additives may be added optionally to a device according to the present invention.

Optional additives preferably comprise one or more additives selected from the group consisting of stabilisers, enhancers and anti-irritants.

Stabilizers may be selected from the group consisting of antioxidants including vitamin E, i e tocopherole, ascorbic acid, sodium pyrosulfite, or butylated hydroxytoluene (BHT), butylated hydroxyanisole, edetic acid and edetate salts; and preservatives including citric acid, tartaric acid, lactic acid, malic acid, acetic acid, benzoic acid, and sorbic acid. Preferred embodiments comprise an antioxidant as the stabiliser, and even more preferably vitamin E and/or butylated hydroxytoluene (BHT).

Enhancers may be selected from the group consisting of
- alcohols, such as short chain alcohols, e.g ethanol and the like, long chain fatty alcohols, e.g. lauryl alcohols, and the like, and polyalcohols, e.g. propylene glycol, glycerin and the like;
- amides, such as amides with long aliphatic chains, or aromatic amides like N,N-diethyl-m-toluamide;
- amino acids;
- azone and azone-like compounds;
- essential oils, i.e. essential oils or constituents thereof, such as 1-carvone, 1-menthone and the like;
- fatty acids and fatty acid esters, such as oleic acid, lauric acid and the like, further esters of fatty acids, such as isopropyl myristate, and various esters of lauric acid and of oleic acid and the like;
- macrocyclic compounds, such as cyclopentadecanone and cyclodextrins;
- phospholipid and phosphate compounds, such as phospholipids;
- 2-pyrrolidone compounds; and
- miscellaneous compounds, like sulphoxides, such as dimethyl sulphoxides, and fatty acid ethers, such as Laureth-9 and polyoxylaurylether.

Combinations of enhancers from different groups may prove useful and efficient.

An example of a useful anti-irritant is vitamin E.

### Examples

The numbers within brackets refer to the corresponding numbers in the figures.

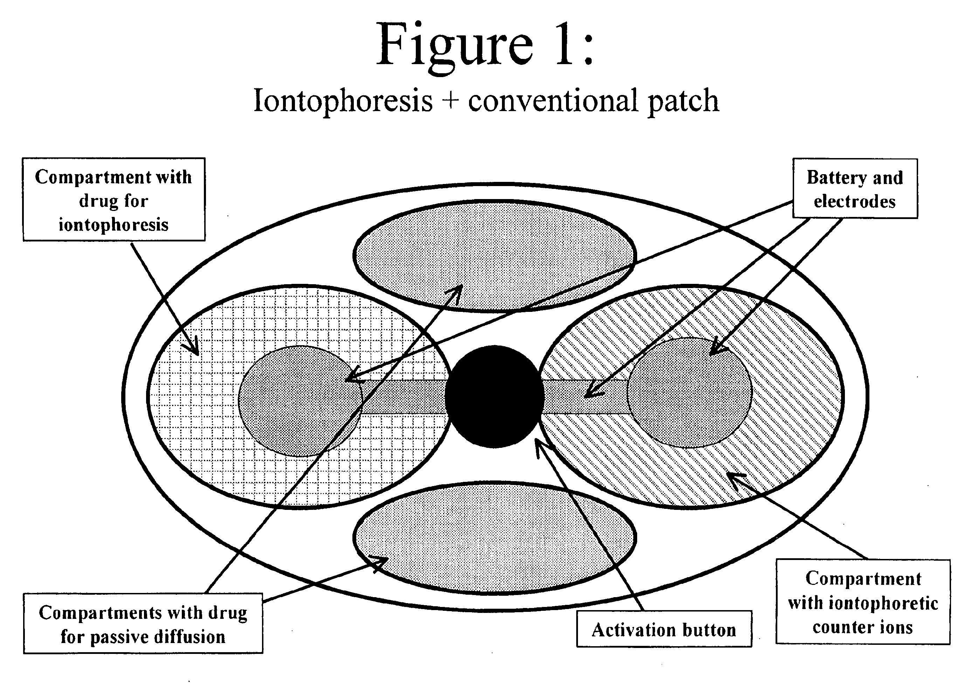

### Example 1, being schematically illustrated in Figure 1.

### Basic administration from matrix part and additional administration from iontophoretic part.

A device in the format of a transdermal patch consisting of two different nicotine delivery systems:

One system where the transdermal dose of nicotine is delivered from a polymeric matrix compartment (11). The mechanism for transdermal nicotine delivery for this system is passive diffusion along a concentration gradient. The rate of delivery is governed by the properties of the matrix polymers and the concentration of nicotine loaded into the matrix. The nicotine in the matrix is in its neutral, base form.

One system where the transdermal dose of nicotine is delivered from a compartment where the mechanism of nicotine transport is iontophoresis, i.e. the driving force is supplied by an electric current (12, 13). In this system positively charged nicotine is loaded in the electrode-fitted (anode) drug compartment (12). The system is completed by a second electrode-fitted (cathode) compartment (13) where negatively charged counter-ions, preferably chloride ions but also other biocompatible negatively charged ions may be used, are loaded. Electrodes (14) are preferably of the Ag/AgCI type. A disposable battery (14), capable of outputting a current of up to 0.5 mA/cm² of drug compartment area in contact with the skin, is connected to the electrodes. When the iontophoretic system is activated, the skin tissue at the application site completes the electrical circuit. The rate of nicotine delivery for this system is governed by the size of the electrical current between the electrodes (14).

The two above systems are placed side-by-side in a thin patch-like device 20-50 cm² in size. The device is fastened at the application site by means of an adhesive layer covering the bottom of the device. The device is backed by a flexible non-woven material, which provides structural support. Directly below the non-woven material is a thin aluminum layer, acting as a barrier to nicotine diffusion through the backing material of the device. The device is also fitted with an activation button (15) to initiate the driving electrical current of the iontophoretic system. The passive diffusion system provides a basic dose of nicotine. The user may activate the iontophoretic system by pressing the activation button (15), and thus release an additional dose of nicotine. Deactivation of the iontophoretic system is by a timer function. Alternatively, the user may at any time deactivate the additional administration of nicotine by pressing the activation button (15) a second time.

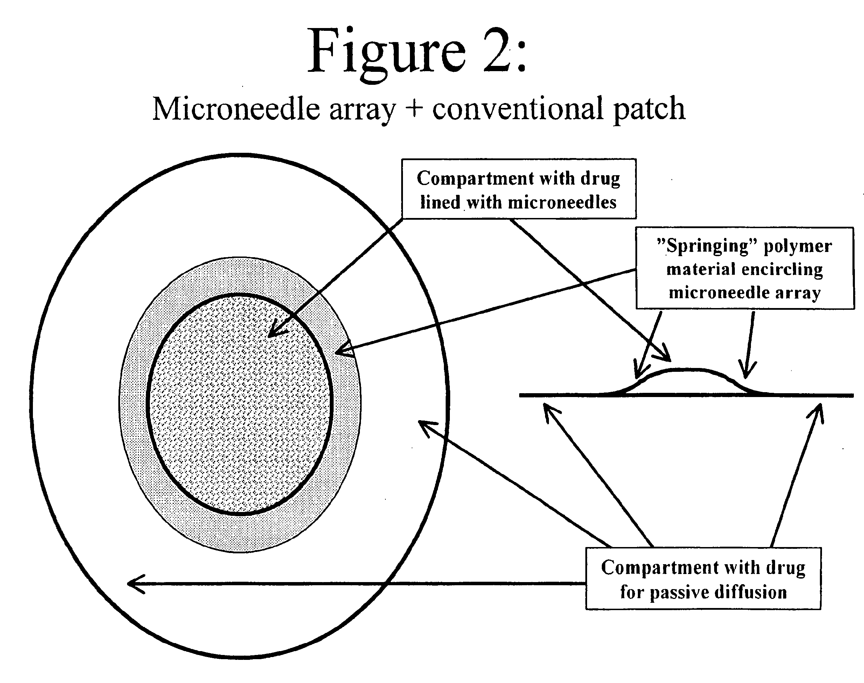

### Example 2, being schematically illustrated in Figure 2.

### Basic administration from reservoir part and additional administration from micro-needle part.

A device in the format of a transdermal patch consisting of two different nicotine delivery systems:

One system where the transdermal dose of nicotine is delivered from a reservoir compartment, through a rate-controlling membrane (21). The mechanism for transdermal nicotine delivery for this system is passive diffusion along a concentration gradient. The rate of delivery is governed by the properties of the rate-controlling membrane and the concentration of nicotine loaded into the compartment. The nicotine in the matrix is in its neutral, base form.

One system where the transdermal dose of nicotine is delivered from a reservoir compartment lined on one side with micro-needles with the function of being able to puncture the stratum corneum of the skin, thus increasing the transdermal delivery rate of nicotine (22). The micro-needles are suspended a short distance from the skin surface and the user may activate the system by pressing on the backing of the patch to bring the micro-needles in contact with the skin. The rate of delivery is governed by the properties of the micro-needles and the concentration of nicotine loaded into the compartment. The nicotine in the micro-needle reservoir compartment is in its neutral, base form.

The two above systems are placed side-by-side in a thin patch-like device 20-50 cm² in size. The device is backed by a flexible non-woven material, which provides structural support. Directly below the non-woven material is a thin aluminum layer, acting as a barrier to nicotine diffusion through the backing material of the device. The device is fastened at the application site by means of an adhesive layer covering the bottom of the device. The immediate area surrounding the micro-needle part of the device consists of a semi-rigid flexing polymer (23) that will allow the micro-needles to flex in and out of contact with the stratum corneum (24) dictated by the user pressing on the backing of the device, directly behind the micro-needles. The passive diffusion system provides a basic dose of nicotine. The user may activate an additional dose of nicotine by pressing on the device backing so that the micro-needles puncture the stratum corneum (24) of the skin and channel nicotine through the stratum corneum (24) to the underlying tissue.

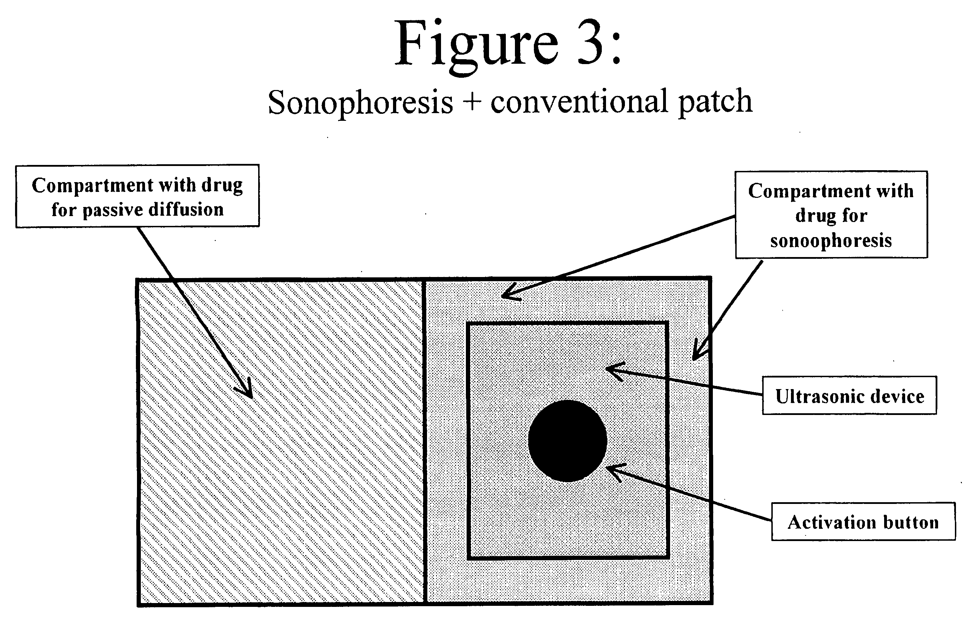

### Example 3, being schematically illustrated in Figure 3.

### Basic administration from matrix part and additional administration from ultrasonic part.

A device in the format of a transdermal patch consisting of two different nicotine delivery systems:

One system where the transdermal dose of nicotine is delivered from a polymeric matrix compartment (31). The mechanism for transdermal nicotine delivery for this system is passive diffusion along a concentration gradient. The rate of delivery is governed by the properties of the matrix polymers and the concentration of nicotine loaded into the matrix. The nicotine in the matrix is in its neutral, base form.

One system where the transdermal dose of nicotine is delivered from a polymeric matrix compartment (32) where the mechanism of nicotine transport is sonophoresis, i.e. an ultrasonic device (33) causes heating of the skin and a reversible disordering of the structure of the stratum corneum. The rate of nicotine delivery for this system is governed by the intensity of the ultrasonic vibrations. The nicotine in the matrix is in its neutral, base form.

The two above systems are placed side-by-side in a thin patch-like device 20-50 cm² in size. The device is fastened at the application site by means of an adhesive layer covering the bottom of the device. The device is also fitted with an activation button (34) to initiate the ultrasonic vibrations. The passive diffusion system provides a basic dose of nicotine. The user may activate the sonophoretic system by pressing the activation button (34), and thus release an additional dose of nicotine. Deactivation of the sonophoretic system is by a timer function. Alternatively, the user may at any time deactivate the additional administration of nicotine by pressing the activation button (34) a second time.

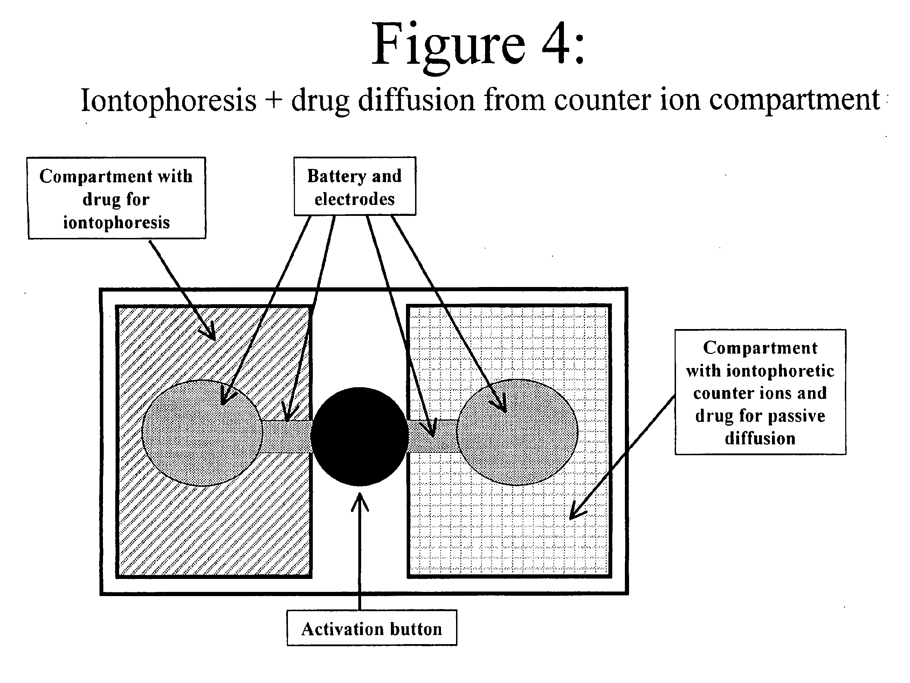

### Example 4, being schematically illustrated in Figure 4.

### Basic administration from matrix part sharing space with iontophoretic part providing additional administration.

A device in the format of a transdermal patch consisting of two different nicotine delivery systems:

One system where the transdermal dose of nicotine is delivered from a compartment where the mechanism of nicotine transport is iontophoresis, i.e. the driving force is supplied by an electric current. In this system positively charged nicotine is loaded in the electrode-fitted (anode) drug compartment (41). The system is completed by a second electrode-fitted (cathode) compartment (42) where negatively charged counter-ions are loaded. This compartment also contains the neutral base nicotine of the passive diffusion system detailed below. A battery (43) supplies the needed electrical power. The rate of nicotine delivery for this system is governed by the size of the electrical current between the electrodes.

One system where the transdermal dose of nicotine is delivered from a polymeric matrix compartment (42). This compartment contains both nicotine in its neutral, base form and the negatively charged counter-ions needed for the iontophoretic system. The mechanism for transdermal nicotine delivery for this system is passive diffusion along a concentration gradient. The rate of nicotine delivery is governed by the properties of the matrix polymers and the concentration of nicotine loaded into the matrix.

The two above systems are fitted in a thin patch-like device 20-50 cm² in size. The device is fastened at the application site by means of an adhesive layer covering the bottom of the device. The device is backed by a flexible non-woven material, which provides structural support. Directly below the non-woven material is a thin aluminum layer, acting as a barrier to nicotine diffusion through the backing material of the device. The device is also fitted with an activation button (44) to initiate the driving electrical current of the iontophoretic system. The passive diffusion part, located in the iontophoretic cathode compartment (42), provides a basic dose of nicotine. The user may activate the iontophoretic system by pressing the activation button (44), and thus release an additional dose of positively charged nicotine from the iontophoretic anode compartment (41). Deactivation of the iontophoretic system is by a timer function. Alternatively, the user may at any time deactivate the additional administration of nicotine by pressing the activation button (44) a second time.

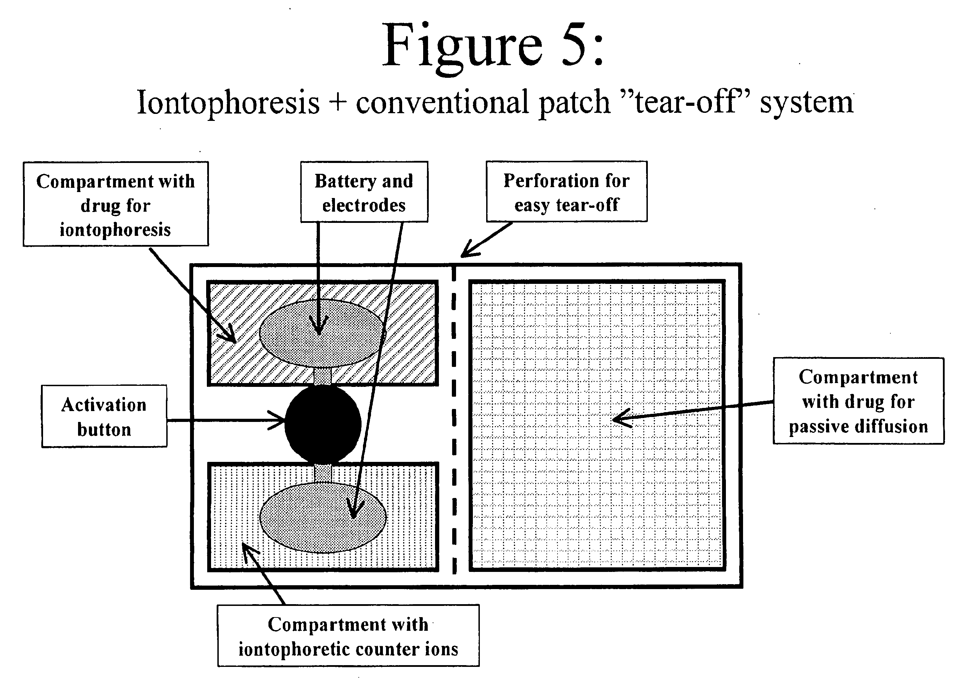

### Example 5, being schematically illustrated in Figure 5.

### Basic administration from reservoir part and additional administration from iontophoretic part, where the two parts may be individually applied to the skin.

A device in the format of a transdermal patch consisting of two different nicotine delivery systems:

One system where the transdermal dose of nicotine is delivered from a reservoir compartment, through a rate-controlling membrane (51). The mechanism for transdermal nicotine delivery for this system is passive diffusion along a concentration gradient. The rate of delivery is governed by the properties of the rate-controlling membrane and the concentration of nicotine loaded into the compartment. The nicotine in the matrix is in its neutral, base form.

One system where the transdermal dose of nicotine is delivered from a compartment where the mechanism of nicotine transport is iontophoresis, i.e. the driving force is supplied by an electric current. In this system positively charged nicotine is loaded in an electrode-fitted drug compartment (52). The system is completed by a second electrode-fitted compartment where negatively charged counter-ions are loaded (53). A battery (54) supplies the needed electrical power. The rate of nicotine delivery for this system is governed by the size of the electrical current between the electrodes.

The two above systems are placed side-by-side in a thin patch-like device 20-50 cm² in size. The device is fastened at the application site by means of an adhesive layer covering the bottom of the device. The device is backed by a flexible non-woven material, which provides structural support. Directly below the non-woven material is a thin aluminum layer, acting as a barrier to nicotine diffusion through the backing material of the device. The two systems are separated by a perforation (55) in the device, making it possible to tear off one system from the other for individual placement on the body of the two parts. The iontophoretic part of the device is also fitted with an activation button (56) to initiate the driving electrical current of the iontophoretic system. The passive diffusion system provides a basic dose of nicotine. The user may activate the iontophoretic system by pressing the activation button (56), and thus release an additional dose of nicotine. Deactivation of the iontophoretic system is by a timer function. Alternatively, the user may at any time deactivate the additional administration of nicotine by pressing the activation button (56) a second time.

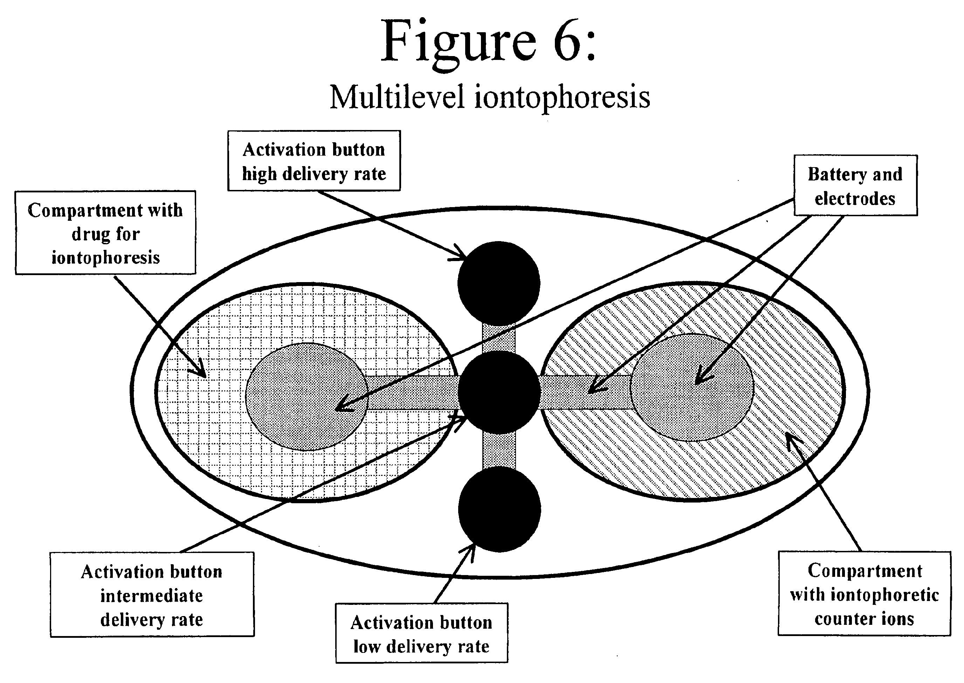

### Example 6, being schematically illustrated in Figure 6.

### Basic administration from matrix part and additional, multilevel administration from iontophoretic part.

A device in the format of a transdermal patch consisting of two different nicotine delivery systems:

One system where the transdermal dose of nicotine is delivered from a polymeric matrix compartment (61). The mechanism for transdermal nicotine delivery for this system is passive diffusion along a concentration gradient. The rate of delivery is governed by the properties of the matrix polymers and the concentration of nicotine loaded into the matrix. The nicotine in the matrix is in its neutral, base form.

One system where the transdermal dose of nicotine is delivered from a compartment where the mechanism of nicotine transport is iontophoresis, i.e. the driving force is supplied by an electric current. In this system positively charged nicotine is loaded in the electrode-fitted (anode) drug compartment (62). The system is completed by a second electrode-fitted (cathode) compartment (63) where negatively charged counter-ions are loaded. A battery (64) supplies the needed electrical power. The rate of nicotine delivery for this system is governed by the size of the electrical current between the electrodes.

The two above systems are placed side-by-side in a thin patch-like device 20-50 cm² in size. The device is fastened at the application site by means of an adhesive layer covering the bottom of the device. The device is backed by a flexible non-woven material, which provides structural support. Directly below the non-woven material is a thin aluminum layer, acting as a barrier to nicotine diffusion through the backing material of the device. The passive diffusion system provides a basic dose of nicotine. The device is also fitted with a set of activation buttons (65, 66, 67) to initiate the driving electrical current of the iontophoretic system. The user may activate the iontophoretic system by pressing one of the activation buttons (65, 66, 67), and thus release an additional dose of nicotine. The amount of nicotine of the additional dose ranges from low to high, depending on which button is pressed by the user. Each button corresponds to a pre-set dose level. Deactivation of the iontophoretic system is by a timer function. Alternatively, the user may at any time deactivate the additional administration of nicotine by pressing the same activation button a second time.

### Further embodiments

The present device should preferably be occlusive.

The present device may deliver nicotine during a predefined period of time, preferably 12, 16, 24 or 48 hours.

The at least one part providing for basic transdermal administration and the at least one part providing for additional and user activatable transdermal administration may be combined in different ways. For example may said parts have at least one feature in common, e g a common adhesive layer and/or a common drug reservoir. Said parts may also be combined so that they have no features in common.

## Claims

1. Device for transdermal administration of nicotine in any form, **characterized in that** it comprises at least one first part providing for basic administration of nicotine in any form and at least one second part providing for additional administration of nicotine in any form, the at least one second part being activatable by the user,
**in that** the at least one first part providing for basic administration is/are (a) transdermal patch(es), preferably of the reservoir type, the matrix type, the drug-in-adhesive type and/or the multi-laminate type, preferably of the drug-in-adhesive type or the reservoir type or combinations of these two types, and
**in that** the at least one second part providing for additional administration comprise(s) means for iontophoretic delivery, sonophoresis, jet injection and/or micro-needles.

2. Device for transdermal administration according to claim 1, **characterized in that** the at least one second part providing for additional administration comprise(s) means for iontophoretic delivery.

3. Device for transdermal administration according to any preceding claim, **characterized in that** the at least one first part and the at least one second part have at least one feature in common.

4. Device for transdermal administration according to any of claims 1 - 3, **characterized in that** the at least one first part and the at least one second part are detachable from one another and/or may be individually applied.

5. Device for transdermal administration according to any preceding claim, **characterized in that** the form of nicotine is selected from nicotine free base, a nicotine salt, such as a tartrate, hydrogen tartrate, citrate, maleate or hydrochloride, a nicotine inclusion complex, such as a complex with a cyclodextrin, a nicotine cation exchanger, such as nicotine with polyacrylate or nicotine in any non-covalent binding, nicotine bound to zeolites, nicotine bound to cellulose or nicotine bound to starch microspheres, and mixtures thereof.

6. Device for transdermal administration according to claim 5, **characterized in that** the form of nicotine is nicotine free base.

7. Device for transdermal administration according to claim 5 or 6, **characterized in that** the least one part providing for basic administration of nicotine is for delivery of nicotine in another form than the at least one part providing for additional administration of nicotine.

8. Device for transdermal administration according to anyone of the preceding claims, **characterized in that** it delivers nicotine during a predefined period of time, preferably 12, 16, 24 or 48 hours.

9. Device according to anyone of the preceding claims, **characterized in that** it further comprises one or more stabilisers, preferably selected from the group consisting of antioxidants including vitamin E, i e tocopherole, ascorbic acid, sodium pyrosulfite, butylated hydroxytoluene (BHT), butylated hydroxyanisole, edetic acid and edetate salts, and preservatives including citric acid, tartaric acid, lactic acid, malic acid, acetic acid, benzoic acid, and sorbic acid, preferably vitamin E and/or butylated hydroxytoluene (BHT); and/or
that it further comprises one or more substances enhancing transdermal penetration, preferably a compound selected from the group consisting of
- alcohols, such as short chain alcohols, e.g ethanol and the like, long chain fatty alcohols, e.g. lauryl alcohols, and the like, and polyalcohols, e.g. propylene glycol, glycerin;
- amides, such as amides with long aliphatic chains, or aromatic amides like N,N-diethyl-m-toluamide;
- amino acids;
- azone and azone-like compounds;
- essential oils, i.e. essential oils or constituents thereof, such as 1-carvone, 1-menthone and the like;
- fatty acids and fatty acid esters, such as oleic acid, lauric acid and the like, further esters of fatty acids, such as isopropyl myristate, and various esters of lauric acid and of oleic acid;
- macrocyclic compounds, such as cyclopentadecanone and cyclodextrins;
- phospholipid and phosphate compounds, such as phospholipids;
- 2-pyrrolidone compounds; and
- miscellaneous compounds, like sulphoxides, such as dimethyl sulphoxides, and fatty acid ethers, such as Laureth-9 and polyoxylaurylether; and/or
that it further comprises one or more substances reducing irritant reactions, preferably vitamin E.

10. Device according to anyone of the preceding claims, **characterized in that** it is occlusive.

11. Use of nicotine for the manufacture of a device for aiding in smoking cessation, in temporary smoking abstinence and/or in reducing the urge to smoke or to otherwise use tobacco containing material, and/or for treating conditions suitable for treatment with nicotine, such conditions being selected from the group consisting of Alzheimer's disease, Crohn's disease, Parkinson's disease, Tourette's syndrome, ulcerous colitis and post-smoking-cessation weight control, such a device being according to anyone of claims 1 - 10.

12. Use according to claim 11 in combination with use of means for administration of nicotine selected from mouth sprays, nasal sprays, transdermal patches, inhaling devices, lozenges, tablets and from parenteral methods, subcutaneous methods, intravenous methods, rectal methods, vaginal methods and transmucousal methods, including use of tobacco.

## Patentansprüche

1. Vorrichtung für die transdermale Verabreichung von Nikotin in irgendeiner Form, **dadurch gekennzeichnet, dass** sie mindestens einen ersten Teil, der die grundlegende Verabreichung von Nikotin in irgendeiner Form zur Verfügung stellt, und mindestens einen zweiten Teil, der die zusätzliche Verabreichung von Nikotin in irgendeiner Form zur Verfügung stellt, umfasst, der mindestens eine zweite Teil ist durch den Verwender aktivierbar,
dass der mindestens eine erste Teil, der die grundlegende Verabreichung zur Verfügung stellt, (ein) transdermale(s) Pflaster ist/sind, bevorzugt die des Reservoirtyps, des Matrixtyps, des Wirkstoff-in-Adhesiv-Typs und/oder des Vielschichtentyps, bevorzugt des Wirkstoff-in-Adhesiv-Typs oder des Reservoirtyps oder eine Kombination dieser beiden Typen, und
dass der mindestens eine zweite Teil, der die zusätzliche Verabreichung zur Verfügung stellt, Mittel für die iontophoretische Abgabe, Sonophorese, Jet-Injektion und/oder Mikronadeln umfasst.

2. Vorrichtung für die transdermale Verabreichung nach Anspruch 1, **dadurch gekennzeichnet, dass** in dem mindestens einen zweiten Teil, der die zusätzliche Verabreichung zur Verfügung stellt, Mittel für die iontophoretische Abgabe umfasst sind.

3. Vorrichtung für die transdermale Verabreichung nach irgendeinem der vorhergehenden Ansprüche, **dadurch gekennzeichnet, dass** der mindestens eine erste Teil und der mindestens eine zweite Teil mindestens ein Merkmal gemeinsam haben.

4. Vorrichtung für die transdermale Verabreichung nach irgendeinem der Ansprüche 1 bis 3, **dadurch gekennzeichnet, dass** der mindestens eine erste Teil und der mindestens eine zweite Teil voneinander abtrennbar sind und/oder einzeln angewendet werden können.

5. Vorrichtung für die transdermale Verabreichung nach irgendeinem der vorhergehenden Ansprüche, **dadurch gekennzeichnet, dass** die Form des Nikotins aus einer freien Nikotinbase, einem Nikotinsalz, wie etwa ein Tartrat, Hydrogentartrat, Citrat, Maleat oder Hydrochlorid, ein Nikotineinschlusskomplex, wie etwa ein Komplex mit einem Cyclodextrin, ein Nikotinkationaustauscher, wie etwa Nikotin mit Polyacrylat oder Nikotin in irgendeiner nicht-kovalenten Bindung, zeolitgebundenes Nikotin, cellulosegebundenes Nikotin, oder stärkegebundene Nikotinmikrokapseln und Mischungen davon ausgewählt ist.

6. Vorrichtung für die transdermale Verabreichung nach Anspruch 5, **dadurch gekennzeichnet, dass** die Nikotinform eine freie Nikotinbase ist.

7. Vorrichtung für die transdermale Verabreichung nach Anspruch 5 oder 6, **dadurch gekennzeichnet, dass** der mindestens eine Teil, der die grundlegende Verabreichung von Nikotin zur Verfügung stellt, für die Abgabe von Nikotin in einer anderen Form vorliegt als der mindestens eine Teil, der die zusätzliche Verabreichung von Nikotin zur Verfügung stellt.

8. Vorrichtung für die transdermale Verabreichung nach irgendeinem der vorhergehenden Ansprüche, **dadurch gekennzeichnet, dass** sie Nikotin während einer vordefinierten Zeitspanne, bevorzugt 12, 16, 24 oder 48 Stunden, abgibt.

9. Vorrichtung für die transdermale Verabreichung nach irgendeinem der vorhergehenden Ansprüche, **dadurch gekennzeichnet, dass** sie ferner einen oder mehrere Stabilisatoren umfasst, bevorzugt die aus der Gruppe, bestehend aus Antioxidantien einschließlich Vitamin E, zum Beispiel Tocopherol, Ascorbinsäure, Natriumpyrosulfit, butyliertes Hydroxytoluol (BHT), butyliertes Hydroxyanisol, Edetinsäure und Edetatsalze und Konservierungsmittel einschließlich Zitronensäure, Weinsäure, Milchsäure, Maleinsäure, Essigsäure, Benzoesäure und Sorbinsäure, bevorzugt Vitamin E und/oder butyliertes Hydroxytoluol (BHT), ausgewählt sind; und/oder
dass sie ferner eine oder mehrere Substanzen umfasst, die das transdermale Eindringen verstärken, bevorzugt eine Verbindung, die aus der Gruppe, bestehend aus
- Alkoholen, wie etwa kurzkettige Alkohole, zum Beispiel Ethanol und dergleichen, langkettige Fettalkohole, zum Beispiel Laurylalkohole und dergleichen und Polyalkohole, zum Beispiel Propylenglykol, Glyzerin;
- Amiden, wie etwa Amide mit langen aliphatischen Ketten oder aromatische Amide, wie N,N-Diethyl-m-toluamid;
- Aminosäuren;
- Azon und azonartige Verbindungen;
- essentielle Ölen, zum Beispiel essentielle Öle oder Bestandteile daraus, wie etwa L-Carvon, L-Menthon und dergleichen;
- Fettsäuren und Fettsäureestern, wie etwa Ölsäure, Laurinsäure und dergleichen, ferner Fettsäureestern, wie etwa Isopropylmyristat und unterschiedliche Ester der Laurinsäure und der Ölsäure;
- makrozyklische Verbindungen, wie etwa Cyclopentadecanone und Cyclodextrine;
- Phospholipid- und Phosphatverbindungen, wie etwa Phospholipide;
- 2-Pyrrolidonverbindungen; und
- diverse Verbindungen, wie Sulfoxide, wie etwa Dimethylsulfoxid und Fettsäureether, wie etwa Laurylether-9 und Polyoxylaurylether, ausgewählt ist; und/oder
dass sie ferner eine oder mehrere Substanzen umfasst, die Reizungen reduzieren, bevorzugt Vitamin E.

10. Vorrichtung nach irgendeinem der vorhergehenden Ansprüche, **dadurch gekennzeichnet, dass** sie verschließend ist.

11. Verwendung von Nikotin für die Herstellung einer Vorrichtung zur Unterstützung der Einstellung des Rauchens, bei zeitlicher Abstinenz vom Rauchen und/oder bei der Verringerung des Drangs zu Rauchen oder Tabak beinhaltendes Material anderweitig zu verwenden, und/oder für die Behandlung von Verfassungen, die sich eignen um mit Nikotin behandelt zu werden, solche Verfassungen, die aus der Gruppe, bestehend aus Alzheimer, Morbus Crohn, Parkinson, Tourette Syndrom, geschwürartige Dickdarmentzündung und Gewichtskontrolle nach der Einstellung des Rauchens ausgewählt sind, so eine Vorrichtung ist eine nach irgendeinem der Ansprüche 1 bis 10.

12. Verwendung nach Anspruch 11 in Verbindung mit der Verwendung von Mitteln für die Verabreichung von Nikotin, ausgewählt aus Mundsprays, Nasensprays, transdermalen Pflastern, Inhalationsvorrichtungen, Lutschtabletten, Tabletten und aus parenteralen Verfahren, subkutanen Verfahren, intravenösen Verfahren, rektalen Verfahren, vaginalen Verfahren und transmukosalen Verfahren, einschließlich der Verwendung von Tabak.

## Revendications

1. Dispositif pour administration transdermique de nicotine sous n'importe quelle forme, **caractérisé en ce qu'**il comprend au moins une première partie fournissant une administration de base de nicotine sous n'importe quelle forme et au moins une seconde partie fournissant une administration supplémentaire de nicotine sous n'importe quelle forme, ladite au moins une seconde partie pouvant être activée par l'utilisateur,
**en ce que** ladite au moins une première partie fournissant une administration de base est/sont (a) un ou des patchs transdermiques, de préférence de type à réservoir, de type à matrice, de type à médicament dans l'adhésif, et/ou de type multicouches, de préférence du type à médicament dans l'adhésif ou de type à réservoir, ou des combinaisons de ces deux types, et
**en ce que** ladite au moins une seconde partie fournissant une administration supplémentaire comprend des moyens pour administration iontophorétique, sonophorèse, injection par jet et/ou micro-aiguilles.

2. Dispositif pour administration transdermique selon la revendication 1, **caractérisé en ce que** ladite au moins une seconde partie fournissant une administration supplémentaire comprend des moyens pour administration iontophorétique.

3. Dispositif pour administration transdermique selon l'une quelconque des revendications précédentes, **caractérisé en ce que** ladite au moins une première partie et ladite au moins une seconde partie ont au moins une caractéristique en commun.

4. Dispositif pour administration transdermique selon l'une quelconque des revendications 1-3, **caractérisé en ce que** ladite au moins une première partie et ladite au moins une seconde partie sont détachables l'une de l'autre et/ou peuvent être individuellement appliquées.

5. Dispositif pour administration transdermique selon l'une quelconque des revendications précédentes, **caractérisé en ce que** la forme de nicotine est choisie parmi une base libre de nicotine, un sel de nicotine, comme un tartrate, un tartrate d'hydrogène, un citrate, maléate ou hydrochlorure, un complexe d'inclusion de nicotine, comme un complexe avec une cyclodextrine, un échangeur de cation de nicotine, comme la nicotine avec un polyacrylate ou la nicotine dans n'importe quelle liaison non-covalente, la nicotine liée aux zéolites, la nicotine liée à la cellulose ou la nicotine liée aux microbilles d'amidon et des mélanges de ceux-ci.

6. Dispositif pour administration transdermique selon la revendication 5, **caractérisé en ce que** la forme de nicotine est une base libre de nicotine.

7. Dispositif pour administration transdermique selon la revendication 5 ou 6, **caractérisé en ce que** ladite au moins une partie fournissant une administration de base de nicotine est destinée à administrer la nicotine sous une autre forme que ladite au moins une partie fournissant une administration supplémentaire de nicotine.

8. Dispositif pour administration transdermique selon l'une quelconque des revendications précédentes, **caractérisé en ce qu'**il fournit de la nicotine pendant un laps de temps prédéfini, de préférence 12, 16, 24 ou 48 heures.

9. Dispositif selon l'une quelconque des revendications précédentes, **caractérisé en ce qu'**il comprend en outre un ou plusieurs stabilisateurs, de préférence choisis dans le groupe constitué d'antioxydants comprenant de la vitamine E, à savoir du tocophérol, de l'acide ascorbique, du pyrosulfite de sodium, de l'hydroxytoluène butylé (BHT), de l'hydroxyanisole butylé, de l'acide édétique, et des sels d'édétate, et des conservateurs comprenant l'acide citrique, l'acide tartrique, l'acide lactique, l'acide malique, l'acide acétique, l'acide benzoïque, et l'acide sorbique, de préférence la vitamine E et/ou l'hydroxytoluène butylé (BHT) ; et/ou
**en ce qu'**il comprend en outre une ou plusieurs substances augmentant la pénétration transdermique, de préférence un composé choisi dans le groupe constitué :
- d'alcools, comme des alcools à chaîne courte, par exemple de l'éthanol et similaire, des alcools gras à chaîne longue, par exemple des alcools de lauryle, et similaire, et des polyalcools, par exemple le propylène glycol, la glycérine ;
- d'amides, comme les amides avec des chaînes aliphatiques longues, ou des amides aromatiques comme le N,N-diéthyl-m-toluamide ;
- d'acides aminés ;
- de l'azone et des composés semblables à l'azone ;
- des huiles essentielles, à savoir des huiles essentielles ou leurs constituants, comme le 1-carvone, l'1-menthone, et similaire ;
- des acides gras et des esters d'acide gras, comme l'acide oléique, l'acide laurique, et similaire, d'autres esters d'acides gras, comme l'isopropyl myristate, et différents esters d'acide laurique et d'acide oléique ;
- de composés macrocycliques, comme le cyclopentadécanone et les cyclodextrines ;
- de composés de phospholipides et phosphate, comme les phospholipides ;
- de composés de 2-pyrrolidone ; et
- de composés divers, comme les sulfoxydes, comme les diméthylsulfoxydes, et des éthers d'acide gras, comme le Laureth-9 et le polyoxylauryléther ; et/ou
**en ce qu'**il comprend en outre une ou plusieurs substances réduisant les réactions irritantes, de préférence la vitamine E.

10. Dispositif selon l'une quelconque des revendications précédentes, **caractérisé en ce qu'**il est occlusif.

11. Utilisation de nicotine pour la fabrication d'un dispositif destiné à aider à arrêter de fumer, à l'abstinence temporaire de fumer, et/ou pour réduire l'envie de fumer ou d'utiliser autrement des matières contenant du tabac, et/ou pour traiter des états adaptés pour un traitement avec de la nicotine, ces états étant choisis dans le groupe constitué de la maladie d'Alzheimer, la maladie de Crohn, la maladie de Parkinson, le syndrome de Tourette, la rectocolite hémorragique, et le contrôle du poids après cessation de fumer, un tel dispositif étant selon l'une quelconque des revendications 1-10.

12. Utilisation selon la revendication 11 en combinaison avec l'utilisation de moyens pour administrer la nicotine, choisis parmi les sprays buccaux, les sprays nasaux, les patchs transdermiques, les dispositifs inhalateurs, les pastilles, les comprimés et par voie parentérale, sous-cutanée, intraveineuse, rectale, vaginale, et transmuqueuse, comprenant l'utilisation du tabac.
